# EUROPEAN PATENT APPLICATION

(11) **EP 1 970 035 A1**
(43) Date of publication of application: **17.09.2008**
(21) Application number: 07104309.5
(22) Date of filing: 16.03.2007
(51) Int. Cl.: A61F 13/15, B65D 85/67

(54) **Feminine hygiene kit**

(71) Applicant: The Procter and Gamble Company, Cincinnati, Ohio 45202 (US)
(72) Inventor: Chang, Tchar-Ruan, 60322, Frankfurt (DE); Jatzlau, Norbert Frank, 91626, Schopfloch (DE); Zimmermann, Joerg, 56307 Dürrholz/Daufenbach (DE); Ureta, Mildred Henriquez, 1203 Geneva (CH)
(74) Representative: Briatore, Andrea

(57) **Abstract**

A product kit for feminine hygiene having a plurality of absorbent articles and a plurality of wipes contained in a single external package. The ratio of the number of absorbent articles and the number of wipes is 1.5:1 and 5:1.

## Description

### FIELD OF THE INVENTION

The present invention relates to feminine hygiene kits. More particularly, the invention relates to improved kits comprising a plurality of disposable absorbent articles along with a plurality of wipes in a given numerical ratio. These kits provide a convenient combination of articles which are sometimes used in conjunction and the selected ratio, being aligned with real statistical usage patterns, allows the user to effectively consume all the items contained in the kit while at the same time avoiding leftovers.

### BACKGROUND OF THE INVENTION

Disposable absorbent articles for absorbing and handling body exudates, such as tampons, sanitary napkins, pantiliners, interlabial pads, diapers, incontinence products and the like are widely known in the art. It has been recognized that changing such absorbent articles can be a hygienic challenge, as portions of the user's body need to be cleaned as well as the hands of the person changing the article may become contaminated. This is perceived as embarrassing and also handling of the soiled article and a new absorbent article for replacement is seriously affected by this.

It is known in the art to use wipes for the cleaning of skin portions, which were soiled with body exudates.

Such wipes are usually made of a soft substrate, typically a non woven material and are oftentimes provided with a moist lotion, which eases cleaning and delivery skin benefits to the skin portion treated therewith. For maintaining the moistness of the wipe these wipes are often packaged in wrappers with barrier properties. Exemplary packaged moistened wipes are disclosed e.g. in U.S. Pat. No. 4,428,477 and in U.S patent application 2007-038197A1. Cleaning wipes are normally packaged in stacks in a common package or individually packaged in sachets.

In some cases it is desirable to provide product kits which combine absorbent articles and wipes in a single product kit which is simpler to use and to store

Kits combining absorbent articles and wipes are known in the art. For example European Patent application EP 637,950 from the Procter & Gamble Company describes individually-packaged absorbent articles in combination with individually-packaged wipes wherein each article is combined with one wipe.

Such combined products are needed by the consumer only in special situations and not all the time. For example, a woman typically needs a cleaning wipe upon changing a sanitary napkin when she is out of home. In other instances women might not feel the same need to have a cleaning wipe with their absorbent article. Therefore, having to buy all the time or full packages with such rather expensive combined articles would be conceived in many instances as either unnecessary, or alternatively would create a storage issue in the woman's bathroom, as she had to store normal as well as combined articles and thus consume more storage space.

U.S. patent 7,144,391 describes kits of feminine hygiene products which include different absorbent articles and wipes inside a box divided in compartments. This solution can be advantageous in some cases, because the user can find several articles at her disposal, just having to store one single product.

A problem with product kits combining different products is that often one of the products runs out while another one is still in part unused, therefore requiring to refill the missing article or to dispose of the item in excess.

The kits of the present invention combine absorbent articles and wipes in a specified ratio. This carefully selected ratio allows the users to have in their kits the adequate number of articles they will need according to the identified normal usage pattern so to effectively use storage space in the bathroom and also requiring the purchase of just one product item.

### SUMMARY OF THE INVENTION

The present invention provides a product kit comprising a plurality of absorbent articles and a plurality of wipes contained in a single external package. The kit is provided such that the ratio between the number of absorbent articles and the number of wipes is between 1.5:1 and 5:1 or between 2:1 1 and 4:1. The present invention may also encompasses a kit, as described above, which is contained in a rigid or semi-rigid box including at least one rigid wall which divides the box into at least a first compartment and a second compartment, wherein at least some of said plurality of absorbent articles are contained in the first compartment and at least some of the plurality of wipes are contained in the second compartment.

### BRIEF DESCRIPTION OF THE DRAWINGS

While the specification concludes with claims particularly pointing out and distinctly claiming the subject matter which is regarded as forming the present invention, it is believed that the invention will be better understood from the following description taken in conjunction with the accompanying drawings, in which:

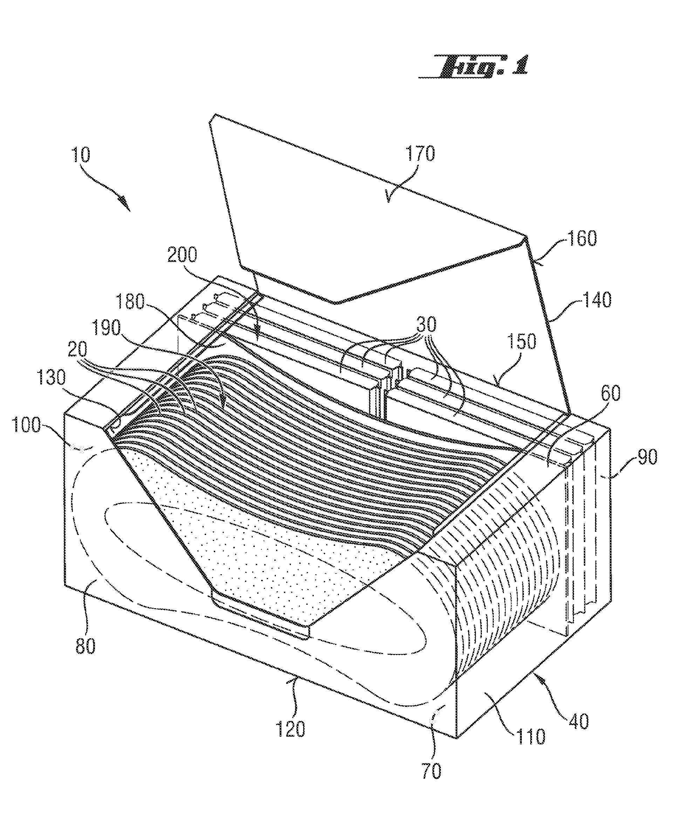

FIG. 1 is a perspective view of a feminine hygiene kit of the present invention.

### DETAILED DESCRIPTION OF THE INVENTION

The term 'absorbent article' is used herein in a very broad sense including any article being able to receive and/or absorb and/or contain and/or retain fluids and/or exudates, especially bodily fluids/bodily exudates. Exemplary absorbent articles in the context of the present invention are disposable absorbent articles such as diapers, incontinence articles and/or feminine hygiene articles. Absorbent articles used in feminine hygiene typically include tampons, light incontinence pads, sanitary napkins, interlabial pads and pantiliners. Tampons are absorbent structure that can be inserted into the vaginal cavity or other body cavities for the absorption of fluid therefrom and are formed by a compressed pledget of absorbent material having, in general, a cylindrical shape. Light incontinence pads, sanitary napkins and pantiliners typically comprise a fluid pervious topsheet as the wearer-facing layer, a fluid impervious backsheet as the garment-facing layer that is some times water vapour and/or gas pervious and an absorbent core comprised therebetween. Furthermore, light incontinence pads, sanitary napkins and pantiliners are normally provided with a means for their attachment to the user's garment, such as, for example, an adhesive..

By 'body fluid' or 'body exudate(s)' it is meant herein any fluid produced by the human body including for instance perspiration, urine, blood, menstrual fluids, vaginal secretions, bowel movement and the like.

The term 'disposable' is used herein to describe articles, which are not intended to be laundered or otherwise restored or reused as an article (i.e. they are intended to be discarded after a single use and preferably to be recycled, composted or otherwise disposed of in an environmentally compatible manner).

The term 'use', as used herein, refers to the period of time that starts when the absorbent article is removed from packaging, if any, and disposed adjacent the wearer's body with the intention of absorbing and/or containing bodily exudates..

The term 'wipe', as used herein, indicates any personal cleaning wipe as known in the art. The wipe may be comprised of a nonwoven fabric which may be comprised of synthetic fibers or natural fibers (such as cellulose) or mixtures thereof. In certain embodiments, the wipe can, for instance, be made of a nonwoven material similar to toilet tissue or facial tissue.

In an embodiment of the present invention the cleaning wipe is moist. Moistening is most typically provided by applying a lotion to the wipe. Suitable lotions are aqueous or non-aqueous ones, being fluid at room temperature. Lotions for use herein increase the cleaning capabilities of the wipe by aiding removal of soiling from the skin. Further, lotions for use herein may also provide skin benefits by reducing friction between the skin and the wipe and / or by containing materials like Aloe vera or chamomile, which increase skin health. Lotions are provided to the wipe in an amount such that the wipe is noticeably moist to the user and that the cleaning capacities of the wipe are increased. Applying lotion to the wipe in an amount such that the lotion drips off from the wipe without exertion of pressure, such as by wringing out, is less desirable herein. Examples for suitable lotions are disclosed in EP-A-808,151; EP-A-763,341 and WO 00/57843.

In some embodiments, the cleaning wipe is both capable of being flushed in a toilet (i.e., it is "flushable"), and may disintegrate sufficiently when flushed in a toilet and when being transported in the sewer system so it does not plug any element of a sewer system. In some embodiments, any wrapping material associated with the wipe can be also flushable and disintegrates. Wrapping materials that meet such criteria include, but are not limited to silicone-treated polyvinyl alcohol films, or films coated with a polyvinyl alcohol, tissue coated or impregnated with polyvinyl alcohol, or similar or other water soluble materials. One material that may be suitable for use as both a cleaning wipe, and as a wrapping for the same is the hydroentangled fabric described in U.S. Pat. No. 4,755,421, issued to Manning, et al. on Jul. 5, 1988.

The size of the cleaning wipe can vary. The cleaning wipe may be greater than or equal to about 25 square centimeters or greater than or equal to about 50 square centimeters in size. The cleaning wipe may be less than or equal to about 1,450 square centimeters in size. In certain embodiments, the cleaning wipe may be between about 100 square centimeters and about 320 square centimeters in size. In one embodiment, the wipe maybe about 225 square centimeters in size.

In certain embodiments, the package of the cleaning wipe may have barrier properties for preventing evaporation of moisture from the cleaning wipe. Suitable barrier materials include laminates, such as laminates where a layer of EVOH (ethylene vinyl alcohol) or PVOH (propylene vinyl alcohol) or aluminium is sandwiched between polymeric film layers, such as, for example, LDPE (low density polyethylene). Further suitable barrier materials for forming the package for the cleaning wipe herein are metallised materials; aluminised materials such as aluminised plastic films; metal foils; oriented polyethylene terephtalate (PET); PETG (glycol-modified PET); oriented polyamide; aromatic polyamide; or polymeric films like polyethylene films with special lacquer coatings, which provide the polymeric film with the vapour barrier functionality. In addition, other materials suitable for this purpose may be used.

An example of a wipe suitable for the present invention has a package material comprising an inner layer comprising polyethylene (PE), an outer layer comprising a copolymer of polyethylene (PE) and polypropylene (PP) and a subjacent intermediate layer comprising polypropylene (PP).

Wipes for use in the present invention may be individually packaged in sachets. Examples of suitable individually packaged wipes are those described in European Patent Application EP 1,752,121.

Figure 1 represents a non limiting embodiment of a product kit according to the present invention. With reference to Fig. 1 the product kit 10 comprises a plurality of absorbent articles 20 and a plurality of wipes 30 contained in a single external package 40. The ratio of the number of absorbent articles 20 to the number of wipes 30 maybe 1.5:1 and 5:1, or between 2:1 and 4:1.

It has been surprisingly found that, despite the fact that typical product kits comprise absorbent articles and wipes in a 1 to 1 ratio, the actual usage ratio for consumers is not 1:1, but rather, between 1.5:1 and 5:1, or between 2:1 and 4:1. These ratios allows the consumers to use up the various articles substantially all at the same time, thus avoiding the need to provide refills of to dispose the articles in excess while still providing the advantages of multiple products inside a single package.

Any external package 40 suitable for a kit according to the present invention may be used. The external package 40 may be manufactured, for example, from cardboard, a polymer bag or film, shrink wrap, or any other suitable material conventionally used for consumer product packaging. Such methods of packing multiple consumer items are generally within the ability of one of ordinary skill in the art. The packing may be selected to maintain clean and hygienic conditions for the individual products. In certain embodiments, it may be desirable for the external package 40 to comprise rigid or semi-rigid materials, such as, for example, plastic and/or cardboard.

In the embodiment of Fig. 1 the kit is contained in an external package 40 which is a cardboard box comprising a top panel 60, a bottom panel 70, a front panel 80, a rear panel 90 a left panel 100 and a right panel 110, wherein the edge 120 between the front panel 80 and the bottom panel 70 defines an horizontal direction and a vertical direction perpendicular to the horizontal direction. The box represented has an opening 130 which is obtained by providing an opening flap, reclosable using known means (140, shown in its open configuration in fig. 1) folding along the edge 150 between the top panel 60 and the rear panel 90 and comprising at least two connected portions (160 and 170 respectively) of the top panel 60 and of the front panel 80 of the box.

As known in the art, absorbent articles can be disposed in the external package 40 either simply in stacks or individually packaged. If individually packaged, the absorbent articles may be packaged in a film wrap and may be folded

In the embodiment shown in Figure 1, the absorbent articles 20 are pantiliners or sanitary napkins disposed inside the box in an unfolded configuration such that their longest dimension is substantially parallel to the horizontal direction. Of course the same configuration can be used within the scope of the present invention to store tampons in the same configuration or individually packaged napkins or pantiliners, both in an unfolded or in a folded configuration.

In embodiments where the plurality of absorbent articles is formed by absorbent articles selected from sanitary napkins or pantiliners, the absorbent articles may include a topsheet, defining a top surface and a backsheet defining a back surface. In these embodiments, the absorbent articles 20 may be positioned inside the box so that their back surfaces are substantially parallel to the front panel. This configuration may facilitate withdrawal of absorbent articles from the kit without the need to touch the topsheet.

In some embodiments, the plurality of wipes 30 may be individually folded and packaged. The wipes may be packaged in sachets, each sachet having a top surface and a back surface. These sachets may be positioned so that their top surface is substantially parallel to the front panel 80 and wherein the minimal distance between the back surface of each absorbent article and the inner surface of the front panel is lower than the minimal distance between the top surface of each wipe sachet and the inner surface of the front panel. In other words, the plurality of absorbent articles may be placed in the front part of the box and the plurality of absorbent articles may be disposed in the back part of the box.

The present invention also encompasses a kit, as described above, which is contained in a rigid or semi-rigid box comprising at least one rigid wall 180 which divides the box in at least a first compartment 190 and a second 200 compartment and wherein at least some of the plurality of absorbent articles 20 are contained in the first compartment 190 and at least some of the plurality of wipes 30 are contained in the second compartment 200.

In certain embodiments, the at least one rigid wall 180 may be affixed or integral to the internal surface of the external package 40. The surface of the at least one said rigid wall 180 may be parallel to the surface of the front panel 80 so as to define a front compartment and a back compartment, said the plurality of absorbent articles 20 may be contained in the front compartment and the plurality of wipes 30 may be contained in the back compartment or vice-versa.

At least a portion of said rigid wall 180 may have a dimension along the vertical direction which is lower than vertical the dimension of the sachets wherein wipes are packaged. In other words, the height of at least a portion of the rigid wall 180 may be lower than the height of the wipe sachets. This may provide an embodiment wherein the wipe sachets, especially if stored in a back compartment of the box, are easier to grasp. In this case it may be desirable for the profile of the rigid wall to follow the profile of the stack of absorbent articles. In certain embodiments, the upper edge of dividing wall 180 may comprise a concave shape to allow easy dispensing of the wipes while maintaining a good division of the wipes and absorbent articles.

The external package 40 may approximate the shape of a rectangular parallelepiped. Such embodiments may provide for easy stacking of the packages.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. A product kit (10) comprising a plurality of absorbent articles (20) and a plurality of wipes (30) contained in a single external package (40), **characterized by** a ratio of the number of absorbent articles (20) and the number of wipes (30) between 1.5:1 and 5:1.

2. A product kit (10) according to claim 1 wherein the absorbent articles (20) are feminine hygienic absorbent articles selected from catamenial tampons, light incontinence pads, sanitary napkins, interlabial pads, pantiliners and combinations thereof.

3. A product kit (10) according to claim 2 wherein said absorbent articles (20) are selected among sanitary napkins, pantiliners and mixtures thereof, said absorbent articles each having a topsheet defining a top surface and a backsheet defining a back surface.

4. A product kit (10) according to claim 3 wherein said absorbent articles (20) are comprised in an unfolded configuration.

5. A product kit (10) according to claim 3 wherein the absorbent articles (20) are pantiliners.

6. A product kit (10) according to any preceding claim wherein the wipes (30) are wet wipes and wherein each wipe is individually folded and packaged in sachets, each sachet having a top surface and a back surface.

7. A product kit (10) according to any preceding claim wherein said external package (40) is a carton or plastic box which is preferably rigid or semi-rigid comprising a top panel (60), a bottom panel (70), a front panel (80), a rear panel (90) a left panel (100) and a right panel (110), wherein the edge (120) between said front panel (80) and said bottom panel (70) defines an horizontal direction and a vertical direction perpendicular to said horizontal direction.

8. A product kit according to claim 7 wherein said carton or plastic box comprises an opening flap (140) folding along the edge (150) between the top panel (60) and the rear panel (90) and comprising at least two connected portions (160 and 170) of the top panel (60) and of the front panel (80) of said box.

9. A product kit (10) according to claims 7 or 8 wherein said plurality of absorbent articles (20) are positioned inside said box so that their back surfaces are substantially parallel to said front panel (80) and facing it, said plurality of wipes are positioned so that the top surface of their sachets is substantially parallel to said front panel (80) and wherein the minimal distance between the back surface of each absorbent article and the inner surface of said front panel (80) is lower than the minimal distance between the top surface of each wipe sachet and the inner surface of said front panel (80).

10. A product kit (10) according to claim 9 wherein said absorbent articles (20) are positioned so that their longest dimension is substantially parallel to said horizontal direction.

11. A product kit (10) according to claims 7 to 10 wherein said box is divided into al least a first compartment (190) and a second compartment (200) and wherein at least some of said plurality of absorbent articles (20) are contained in said first compartment (190) and at least some of said plurality of wipes (30) are contained in said second compartment (200), said compartments being defined and separated by at least one rigid or semi-rigid wall (180).

12. A product kit (10) according to claim 11 wherein said at least one rigid wall (180) is affixed or integral to the internal surface of said external package (40).

13. A product kit (10) according to claims 11 or 12 wherein the surface of at least one said rigid wall (180) is parallel to the surface of said front panel (80) so to define a front compartment and a back compartment.

14. A product kit (10) according to claim 13 wherein said plurality of absorbent articles (20) is contained in said front compartment and said plurality of wipes (30) is contained in said back compartment.

15. A product kit (10) according to claims 11 to 14 wherein at least a portion of said rigid wall (180) has a dimension along said vertical direction which is lower than the vertical the dimension of said sachets wherein wipes are packaged.
